# EUROPÄISCHE PATENTSCHRIFT

(11) **EP 2 596 408 B1**
(45) Veröffentlichungstag und Bekanntmachung des Hinweises auf die Patenterteilung: **30.04.2014**
(21) Anmeldenummer: 10752270.8
(22) Anmeldetag: 22.07.2010
(51) Int. Cl.: G05B 19/042, G08B 21/04

(54) **SYSTEM UND VERFAHREN ZUM VERARBEITEN VON VISUELLER, AUDITIVER, OLFAKTORISCHER UND/ODER HAPTISCHER INFORMATION**
SYSTEM AND METHOD FOR PROCESSING VISUAL, AUDITORY, OLFACTORY, AND/OR HAPTIC INFORMATION
SYSTÈME ET PROCÉDÉ DE TRAITEMENT D'INFORMATIONS VISUELLES, AUDITIVES, OLFACTIVES ET/OU HAPTIQUES

(43) Veröffentlichungstag der Anmeldung: 29.05.2013
(73) Patentinhaber: Gira Giersiepen GmbH & Co. Kg, 42477 Radevormwald (DE)
(72) Erfinder: KEMMANN, Harald, 42555 Velbert (DE); NAWROCKI, Matthias, 41470 Neuss (DE)
(74) Vertreter: Tönhardt, Marion
(86) Internationale Anmeldenummer: PCT/DE2010/000859
(87) Internationale Veröffentlichungsnummer: WO 2012/010109

(56) Entgegenhaltungen:
- US-A1- 2002 171 551
- US-A1- 2003 058 111
- US-A1- 2006 024 020
- US-A1- 2009 124 863

## Beschreibung

Die Erfindung betrifft ein System und ein Verfahren zum Verarbeiten von visueller, auditiver, olfaktorischer und/oder haptischer Information für das kognitive Bestimmen wenigstens eines Zustandes mindestens eines Raumes und/oder mindestens einer Person und/oder mindestens eines Gegenstandes und zum Finden und Umsetzen einer Entscheidung abhängig von dem wenigstens einen erkannten Zustand.

Komplexe Gebäudetechnik ermöglicht es, eine Vielfalt von Funktionen vorwiegend innerhalb des Hauses mit Hilfe eines Servers zu kontrollieren und zu steuern. Die Möglichkeiten für die Eingabe von Befehlen erstrecken sich dabei von konventionellen Schaltern und Tastern bis hin zum Touchdisplay, gegebenenfalls über eine Fernsteuerung. Neben einem hohen Maß an Komfort, Sicherheit und Flexibilität ist auch immer die Wirtschaftlichkeit in Betracht zu ziehen. Realisiert werden heutzutage beispielsweise eine zentrale Steuerung der Haustechnik, zeitabhängige Temperaturreglung, automatische Lichtsteuerung und vieles mehr.

Der Komfort bei einem System für die Gebäudetechnik würde deutlich gesteigert, wenn das System selbstständig intelligent erkennen würde, ob eine bestimmte Maßnahme gewünscht wird oder eine solche mehr oder weniger zwingend erforderlich ist. Während das letztere Problem mit einer entsprechenden Anzahl geeigneter Sensoren weitgehend bewältigt werden kann, fehlen derzeit Lösungen für ein kognitives System.

Die demografische Entwicklung zeigt zudem, dass es immer mehr ältere Menschen gibt, die so lange wie möglich weitgehend selbstbestimmt in ihrer vertrauten Umgebung leben möchten. Eine ständige Unterstützung durch Freunde oder Verwandte ist nicht immer gegeben, professionelle Unterstützung durch Pflegepersonal ist kostenintensiv und vielfach kaum finanzierbar. Körperliche, aber auch geistige Gebrechen machen viele einfache Tätigkeiten des Alltags bis hin zum Betätigen eines entfernt befindlichen Lichtschalters schwierig oder unmöglich.

Es ist daher die Aufgabe der Erfindung, ein System sowie ein Verfahren der eingangs genanten Gattung zur Verfügung zu stellen, die Menschen bei der Bewältigung ihres Alltags unterstützen und für einen größtmöglichen Komfort sorgen. Anomale Situationen oder bedrohliche Situationen sollen möglichst automatisch erkannt und gemeldet werden.

Diese Aufgabe wird durch ein System nach Anspruch 1 und durch ein Verfahren nach Anspruch 6 gelöst. Vorteilhafte Ausgestaltungen sind Gegenstand der jeweils rückbezogenen Unteransprüche.

Erfindungsgemäß sind bei einem System zum Verarbeiten von visueller, auditiver, olfaktorischer und/oder haptischer Information für das kognitive Bestimmen wenigstens eines Zustandes mindestens eines Raumes und/oder mindestens einer Person und/oder mindestens eines Gegenstandes und zum Finden und Umsetzen einer Entscheidung abhängig von dem wenigstens einen erkannten Zustand ein erstes Erkennungsmodul, das zum Feststellen mindestens der Anwesenheit und gegebenenfalls zum Identifizieren von Personen und/oder Gegenständen ausgelegt ist und ein entsprechendes elektrisches Signals ausgibt, ein Satz Sensoren, wobei der Satz Sensoren dazu ausgelegt ist, visuelle, auditive, olfaktorische und/oder haptische Information quantitativ oder qualitativ aufzunehmen und entsprechende elektrische Signalen auszugeben, und eine Auswertelogik zum Verarbeiten der zumindest vom ersten Erkennungsmodul und von dem Satz Sensoren ausgegebenen Signale vorgesehen, wobei das System weiter vorsieht, dass für den mindestens einen Raum eine zwei- oder dreidimensionale Karte zur Verfügung gestellt ist, welche eine oder mehrere statische Zonen aufweist, wobei jede statische Zone ein Gebiet des Raumes kennzeichnet, aus dem visuelle, auditive, olfaktorische und/oder haptische Information über den Zustand des Raumes, einer Person und/oder eines Gegenstandes zu erlangen ist, eine Einrichtung vorgesehen ist, welche, sobald das erste Erkennungsmodul die Anwesenheit einer oder mehrerer Personen in dem Raum signalisiert, für zumindest eine dieser Personen eine dynamische Zone definiert, aus welcher Bewegungsinformation von der oder über die Person zu erlangen ist, und dadurch gekennzeichnet ist, dass ein zweites Erkennungsmodul vorgesehen ist, das zum Detektieren von Bewegungen einer Person ausgelegt ist, wobei das zweite Erkennungsmodul Information lediglich aus einer dynamischen Zone erhält, und dass die Auswertelogik einen Zustand des Raumes und/oder einer Person und/oder eines Gegenstandes aus den verarbeiteten Signalen erkennt, wobei zum Erkennen des Zustandes an visueller Information nur solche verwendet wird, die aus einer der Zonen herrührt, und eine Entscheidung trifft, basierend auf dem erkannten Zustand, ob eine Aktion einzuleiten ist oder nicht.

Die US 2006/0024020 A1 beschreibt ein videobasiertes Überwachungssystem mit einem Sensor, der visuelle Informationen aufnimmt, wobei das Sichtfeld des Sensors in eine Anzahl von Zonen aufgeteilt ist. Dabei werden beispielsweise Bettzonen oder Türzonen definiert, die auf bestimmte Ereignisse hin überwacht werden und als statische Zonen anzusehen sind. In Aktivitätszonen werden Bewegungen eines Patienten überwacht, beispielsweise die Dauer der Bewegung und die Anzahl der Bewegungen, wobei auch diese Zone als statisch anzusehen ist, da sie vorab festgelegt und definiert wird. Gleiches gilt für die Hoch-Risiko-Zonen, die vorab festgelegt sind, beispielsweise in der Nähe eines Fensters, eines Ofens oder irgendeiner Gefahrenquelle.

Die US 2009/0124863 A1 beschreibt ein System beziehungsweise ein Verfahren, mit dem der Status eines Patienten automatisch aufgezeichnet wird. Dabei werden insbesondere die Gesten überwacht, um zu detektieren, ob der Patient möglicherweise an Schmerzen leidet. Dazu können auch der Gesichtsausdruck und Lautäußerungen des Patienten in Betracht gezogen werden.

Die US 2002/0171551 A1 beschreibt ein Alarmsystem, das den Zustand einer Person, die der Überwachung bedarf, beispielsweise einer älteren Person, die allein zuhause lebt, überwacht. Dazu werden viele unabhängige Signale aufgenommen und kombiniert, um Hinweise zu entdecken, ob ein Eingreifen erforderlich ist. Das System ist ein lernendes System, das auf der Bayes-Statistik beruht.

Ebenso beschreibt die US 2003/0058111 A1 ein lernendes Verfahren, mit dem das Verhalten einer Person beurteilt werden kann.

"Visuelle Information" umfasst dabei Information, die üblicherweise von einer Schwarz-Weiß-Kamera einer Farbkamera oder einer Infrarot-Kamera eingefangen wird. Die visuelle Wahrnehmung stellt den wichtigsten aller menschlichen Sinne dar. Der Mensch ist in der Lage, innerhalb von Bruchteilen einer Sekunde beispielsweise in einem Raum nicht nur quantitativ die Anzahl der Personen zu erfassen, sondern auch qualitativ zwischen einem Kind, einem Tier und einem Erwachsenen zu unterscheiden und zwischen einer Sitz-, einer Stand- und einer Liegeposition zu unterscheiden. Ferner ist er fähig abzuschätzen, ob jemand schläft oder aktiv ist, und assoziiert beispielsweise einen mitten im Raum liegenden, reglosen Menschen direkt mit einer "anomalen" und bedrohlichen Situation.

"Auditive Information" umfasst Information, die aus Schallwellen herrührt, ein geeigneter Sensor ist beispielsweise ein Mikrofon. Dieses kann prüfen, ob sich eine Präsenz im Raum befindet, die sich akustisch bemerkbar macht. Beispielsweise besteht die Möglichkeit, gewünschte Töne aufzunehmen und diese als zusätzlichen Filter einzusetzen. So erzeugt ein Hund beim Laufen andere Geräusche als ein Kind. Der Benutzer kann nun beliebige Geräuschkulissen aufnehmen - beispielsweise das Laufgeräusch eines Hundes - und wählen, ob und wie das System auf dieses Geräusch reagiert. Grundsätzlich lässt sich das Mikrofon ebenfalls zur Spracheingabe nutzen. Die Sprachsteuerung ist allerdings nur in sehr eingeschränkter Form nutzbar, wenn aufgrund der Positionierung des Mikrofons im Gehäuse und des schwankenden Geräuschpegels zum Mikrofon hin keine ausreichend konstante "Sprachqualität" gewährleistet werden kann - überdies ist eine große Distanz des Mikrofons zum Sprecher nachteilig.

"Olfaktorische Information" umfasst Information über Gerüche, mit deren Hilfe beispielsweise die Anwesenheit von Erdgas oder Rauch festgestellt werden kann. Entsprechende Sensoren stehen zur Verfügung. Beispielsweise sind Sensoren, die auf leitenden Polymeren basieren, in der Lage, eine Vielzahl von Gerüchen zu erkennen.

"Haptische Information" umfasst im Wesentlichen Information über Temperatur und Luftfeuchtigkeit. Zwar hat der Mensch kein Sinnesorgan zur Wahrnehmung von Luftfeuchtigkeit. Er empfindet aber individuell das Zusammentreffen von Lufttemperaturen mit bestimmten Werten der Luftfeuchtigkeit als behaglich oder unbehaglich. Die Temperatursensoren sollten dabei so angeordnet werden, dass das Temperaturprofil im Raum hinreichend gut wiedergegeben werden kann. Beispielsweise ist es denkbar, in der Nähe von Türen und Fenstern zusätzliche Temperatursensoren anzuordnen, die die Information eines "zentralen" Sensors ergänzen und mit dem System kommunizieren. Eine weitere Möglichkeit besteht darin, mittels einer Infrarotkamera thermografische Aufnahmen zu erzeugen, um beispielsweise bei einem Menschen einen Unterkühlungszustand oder einen Überhitzungszustand festzustellen. Andere haptische Information, insbesondere taktile Information, steht bei der Erfindung nicht im Vordergrund.

Die Information kann qualitativ oder quantitativ aufgenommen werden. Beispielsweise genügt es, wenn als olfaktorische Information das Vorhandensein eines Gases festgestellt wird, wobei die Konzentration unwichtig ist und deswegen nicht gemessen wird. Information über die Temperatur quantitativ zu erfassen, ist dagegen sehr sinnvoll.

Eine Auswertelogik wertet die Information aus bzw. verarbeitet die ausgegebenen Signale mit Hilfe eines Prozessors, der in üblicher Weise auf Speicher zugreifen kann. Diese Speicher, als nicht-flüchtige Speicher und auch als flüchtige Speicher ausgebildet, enthalten die zum Betrieb des Systems notwendige Software, außerdem Datenbanken und/oder Tabellen, mit deren Hilfe die erlangte Information ausgewertet wird, um einen Zustand zu erkennen, sowie ein Regelwerk, mit dessen Hilfe Entscheidungen gefällt werden können.

Insgesamt wird ein System zur Verfügung gestellt, mit dem die menschlichen Sinnesorgane im Wesentlichen nachgestellt werden können, so dass die Wahrnehmung und Sinnesmodalität eines Menschen simuliert werden. Damit wird es möglich, den Zustand eines Raumes, einer Person oder eines Gegenstandes zu erkennen und basierend darauf fundamentale kognitive Entscheidungen zu treffen, die menschlichen Entscheidungen in demselben Umfeld weitgehend entsprechen würden.

Wesentlich ist dabei, dass die Information, die für die zu treffende Entscheidung notwendig ist, so effizient wie möglich gesammelt wird. Dies ist die Grundlage des Gedankens, das Gebiet des Raumes in statische Zonen aufzuteilen. Statische Zonen in einem Wohn- und Arbeitszimmer können beispielsweise ein den Schreibtischstuhl umgebendes Gebiet, ein das Sofa umgebendes Gebiet, ein ein Fenster umgebendes Gebiet oder ein eine Tür umgebendes Gebiet sein. Lediglich diese statischen Zonen werden bezüglich visueller Information ausgewertet, um Änderungen zu erkennen. Es lässt sich dann feststellen, ob beispielsweise eine Person auf dem Schreibtischstuhl sitzt oder nicht, ob das Sofa besetzt ist oder ob dort vielleicht ein Buch oder ein Laptop liegt. Insbesondere kann damit ermittelt werden, ob ein Fenster oder beispielsweise eine Terrassentür geöffnet, teilweise geöffnet oder geschlossen sind. Dieses lässt sich nicht nur daran feststellen, dass beispielsweise zwischen Zarge und Fensterrahmen bzw. Türrahmen ein Spalt entsteht, sondern ganz einfach auch anhand der Stellung des Fenstergriffes bzw. des Türgriffes. Soll nun als eine Zone beispielsweise das Gebiet um einen Fenstergriff festgelegt werden, so kann aus den Änderungen, die in der Zone stattfinden, abgeleitet werden, ob sich das Fenster in einer Offenstellung, in einer Kippstellung oder in einer Schließstellung befindet. Ergänzend kann natürlich immer noch weitere Information herangezogen werden. Diese statischen Zonen sind benutzerdefinierte Zonen, die bei Inbetriebnahme des Systems definiert werden. Sie lassen sich ändern oder anpassen, wenn sich die räumliche Situation ändert oder wenn sich das Anforderungsprofil ändert. "Statisch" bedeutet insoweit, dass die Zone in dem Raum fest definiert ist und unbeeinflusst von Zustandsänderungen des Raumes, von Gegenständen oder Personen bleibt.

Eine besondere Qualität hat die erfindungsgemäß vorgesehene dynamische Zone, die im Regelfall für Personen definiert wird, und zwar insbesondere für Personen, deren Zustand erkannt werden soll. Dieser Zustand kann zum Beispiel eine Notsituation sein, in die die Person durch einen Unfall oder eine Ohnmacht gebracht worden ist, oder aber ein Zustand, bei dem eine Person durch ihr eigenes Verhalten Situationen beeinflusst. Dabei kommt vor allem die Gestiksteuerung in Betracht. Die Auswertelogik wird dabei aus den Bildänderungen in der dynamischen Zone auf Bewegungen der Person rückschließen und "erlernte" Gesten erkennen und entsprechende Aktionen einleiten. Die dynamische Zone kann aus den Umrissdaten allein oder in Kombination mit Bewegungsdaten abgeleitet werden. Beispielsweise kann der Umriss um einen Bereich erweitert werden und mit diesem die dynamische Zone bilden. In einer solchen erweiterten dynamischen Zone kann eine Lageänderung einer Person dadurch detektiert werden, dass eine Umrissveränderung erfasst wird, wobei beispielsweise bei einem Sturz die Umrissfläche oder "Masse" der Person gegenüber einer stehenden oder sitzenden Haltung plötzlich vergrößert ist.

Hier unterscheidet sich die Erfindung ganz wesentlich vom Stand der Technik, wie er in der WO 03/056882 A1 beschrieben ist. Dort wird offenbart, wie die Beleuchtung in einem Raum gesteuert wird, indem Benutzeraktivitäten beobachtet bzw. berücksichtigt werden. Dabei wird ein vordefiniertes Verhalten eines Benutzers fest mit durchzuführenden Aktionen verknüpft, und dieses festgelegte Regelwerk wird verwendet, wenn bestimmte Situationen auftreten. So wird beispielsweise ein Leselicht eingeschaltet, sobald ein Benutzer in einem definierten Sessel sitzt. Die Erfindung leistet auch dies, geht aber weit darüber hinaus, wie hiernach noch erläutert werden wird.

Mit der Definition von Zonen wird es möglich, einen Großteil der Intelligenz, die ein System eigentlich besitzen müsste, in eine Vorbereitungsphase zu verlagern, so dass das System selbst nur wenig Information verarbeiten muss. Dies macht es möglich, relativ kleine Prozessoren in der Auswertelogik zu verwenden.

Für die Aufteilung der Zonen haben sich bestimmte Grenzen als zweckmäßig herausgestellt. Die minimale Größe einer Zone sollte 3 % des gesamten Bildbereichs betragen, es ist möglich, dass sich einzelne Zonen überschneiden, aber maximal nur zu 10 %. Es hat sich gezeigt, dass maximal 15 Zonen mit vertretbarem Aufwand zu realisieren sind. Eine dynamische Zone nimmt dabei eine Sonderstellung ein, da sie grundsätzlich mit jeder anderen Zone zeitweilig überlappen kann.

Für bestimmte Situationen kann es zweckmäßig sein, temporäre statische Zonen abhängig von einer Bedingung zu erzeugen. Wenn beispielsweise die visuelle Information aus einer statischen Zone ergibt, dass der Fenstergriff in der Offenstellung ist, kann das System eine vorab festgelegte, aber normalerweise nicht abgefragte temporäre statische Zone um den Fensterrahmen aktivieren und auf einen Spalt zwischen Fenster und Rahmen überprüfen. Sollte kein Spalt vorliegen, ist das Fenster (versehentlich) zugeschlagen, und das System gibt eine entsprechende Meldung aus. Ein Satz von Bedingungen für das Erzeugen zugeordneter temporärer statischer Zonen wird vorab im System abgelegt. Dieses Konzept kann generalisiert werden durch
- Festlegen eines Satzes von Bedingungen;
- Definieren einer oder mehrerer temporärer statischer Zonen für jede Bedingung aus dem Satz von Bedingungen;
- Prüfen, ob mindestens eine Bedingung eingetreten ist; und
- Aktivieren der temporären, statischen Zone(n), die für die mindestens eine Bedingung definiert ist; und
- Erlagen von visueller Information aus der/den temporären, statischen Zone(n).

In dem oben geschilderten Umfeld wird ein erfindungsgemäßes System zweckmäßigerweise jeweils nur einem Raum zugeordnet, wobei, abhängig von den Gegebenheiten natürlich die Zuordnung zu mehreren Räumen nicht ausgeschlossen ist. Sofern aber mehrere erfindungsgemäße Systeme in einem Gebäude mit mehreren Räumen verwendet werden, so sollten die einzelnen Systeme in der Lage sein, untereinander zu kommunizieren, um so beispielsweise die Positionen von Personen oder Gegenständen abgleichen zu können.

Nach einer bevorzugten Ausgestaltung des Systems stellt das erste Erkennungsmodul den Umriss einer Person oder eines Gegenstandes fest und bestimmt deren/dessen Identität anhand des Umrisses. Eine solche Vorgehensweise ist bekannt und beispielsweise in der EP 1 574 986 B 1 beschrieben. Hier ist bei einer Vorrichtung zur Personendetektion und zum Personentracking vorgesehen, eine Kameraeinheit über einer zu untersuchenden Fläche anzubringen und die strukturellen Formmerkmale von Personen, wie beispielsweise Kopf- oder Schultergeometrie und Symmetrien zu erkennen und auszuwerten. Dabei wird das Vorliegen geometrischer Merkmale anhand typischer Umrisslinien geprüft. Insbesondere werden die Personen durch ihre Kopfellipsen und die Schulterellipsen detektiert. Diese sind personenspezifisch, so dass auch unterschiedliche Personen entsprechend detektiert werden können. Somit können unterschiedlichen Personen jeweils ihre spezifische dynamische Zone zugeordnet werden.

Obwohl dieses Verfahren grundsätzlich bei dem System gemäß der vorliegenden Erfindung anwendbar ist, wird gemäß einer bevorzugten Ausführungsform ein anderer Weg gewählt. Bei dem hauptsächlich geplanten Einsatzbereich wird es darum gehen, nur eine einzige oder wenige Personen, und zwar immer dieselben, beispielsweise eine Familie, in ihrem Umfeld zu beobachten. So reicht hier oftmals die Unterscheidung "Erwachsener", "Kind" und "Tier" zur Identifikation aus. Diese Unterscheidung kann anhand der durch die Umrisslinien vermittelten Flächen oder "Massen" getroffen werden. Dieser vereinfachte Ansatz macht es möglich, das rohe Datenmaterial durch schnelle und zuverlässige Algorithmen zur Erkennung und Differenzierung von sich bewegenden, Objekten in Echtzeit zu verarbeiten. Zusätzlich zur Bewegungserkennung und Verfolgung eines Objekts kann eine an sich bekannte Technologie eingesetzt werden, die Bereiche des Bildes analysiert, welche sich nicht ändern. Das Hintergrundbild des Kamerabereiches wird untersucht und mit Hilfe eines Lemalgorithmus im Laufe der Zeit formatiert und angepasst. So lernt das erfindungsgemäße System mit der Zeit, Änderungen im Hintergrund als zustandsrelevant oder nicht zustandsrelevant zu beurteilen. Einfallende Sonnenstrahlen in einem Zimmer sollen beispielsweise in Verbindung mit dem Abgleich einer Datenbank mit den Zeiten von Sonnenaufgang und Sonnenuntergang erkannt und als zulässige Bildveränderung, die den Zustand der Person nicht beeinflusst, gewertet werden.

Auch Gegenstände können mit Hilfe der geschilderten Techniken erkannt und identifiziert werden, wenn ihr Umriss eindeutig ist.

Diese Vorgehensweise ist drastisch demgegenüber vereinfacht, was beispielsweise in der DE 20 2007 008 363 U1 beschrieben ist. Die hier zur Beleuchtungssteuerung dienende Vorrichtung detektiert die Bewegung und/oder die Anwesenheit einer Person in einem Erfassungsbereich durch eine digitale elektronische Bilderfassung des Erfassungsbereiches, wobei ein Detektionssignal zur Aktivierung der Beleuchtungseinheit dann erzeugt wird, wenn ein erstes elektronisches Bildsignal eines ersten erfassten digitalen Bildes zumindest einem Bild-Teilbereich verschieden von einem zweiten elektronischen Bildsignal eines zweiten, zeitlich beabstandet erfassten digitalen Bildes bezogen auf den ersten Bild-Teilbereich ist, wobei also immer eine volle Auswertung durchgeführt wird.

Das erfindungsgemäße System wird mit elektrischen und elektronischen Komponenten realisiert, die sich auf einer Platine mit den Abmessungen 10 cm x 10 cm oder weniger unterbringen lassen. Diese Platine kann in einem Gehäuse untergebracht und an einer zweckmäßigen Position in dem zu überwachenden Raum angebracht werden.

Zentrale Komponente für die Beschaffung der visuellen Information ist eine einzige Kamera, die den gesamten Raum und damit alle Zonen abbildet. Vorzugsweise wird eine Kamera mit Fischaugenobjektiv eingesetzt. Die für Fischaugenobjektive typischen Verzerrungen werden durch einen einmaligen Kalibrierprozess vor der Installation der Hardware korrigiert. Dieser Kalibrierprozess ist an sich bekannt und soll hier nicht weiter erläutert werden. Zweckmäßigerweise ist die Kamera mittig auf der Platine angeordnet. Durch das Fischaugenobjektiv entfällt außerdem die Notwendigkeit, die Kamera beweglich zu gestalten. Kontrastverbesserungen können auf übliche Art vorab vorgenommen werden.

Im Sichtbereich der Kamera können sich eine oder mehrere Personen bewegen. Für jede der Personen werden jeweils die Koordinaten im Raum bestimmt. Für den Fall, dass zwei oder drei Personen gleicher Größe und mit gleichem Gewicht und/oder gleicher Kleidungsfarbe hintereinander vor der Kamera stehen, wird die Erkennung auf ein einzelnes ganzes Objekt begrenzt, d.h. die Personen werden nicht unterschieden.

Die Kamera dient nicht nur dazu, Personen oder Gegenstände zu erkennen und zu identifizieren. Vielmehr sollen auch Bewegungen wahrgenommen werden, wobei die Auswertelogik aus Information über die Bewegung durchzuführende Aktionen ableiten soll.

So ist beispielsweise denkbar, zu schaltende Objekte innerhalb des Raumes durch Gestiken zu betätigen, also durch Zeigen anstatt durch Berühren eines Tasters, eines Touchscreens oder dergleichen. Dem Benutzer bleibt es lediglich, Gesten so ausdrucksstark durchzuführen, dass sie von dem System erkannt werden. So kann ein in eine Richtung weisender ausgestreckter oder nahezu ausgestreckter Arm eine Geste definieren, mit der beispielsweise auf einen Lichtschalter gezeigt wird, woraufhin dann die angeschlossene Lichtquelle geschaltet wird. Die Geste kann ebenso direkt auf die Lichtquelle gerichtet werden, auf den Fernseher, einen Computer usw.

Die Auswertelogik kann ebenso bestimmte Handlungen daraus ableiten, dass eine Bewegung nicht vorhanden ist. So kann das System als Sturzmelder arbeiten, das dann Alarm gibt, wenn eine erkannte und identifizierte Person ihre Lage über einen vorbestimmten Zeitraum nicht verändert, insbesondere, wenn sie regungslos auf dem Boden liegt.

Davon unterschieden werden kann eine Schlafsituation, bei der eine Person in der statischen Zone "Bett" ruht. Auch hier können Bewegungen detektiert werden, ergänzend mit Hilfe von Infrarot-Sensoren, wobei aber die Zeiträume, während der keine Bewegung erfolgt, durchaus länger sein dürfen.

Die Erfindung ermöglicht es auch, im Rahmen einer Objektivüberwachung Gegenstände oder Bereiche sozusagen zu tabuisieren. Solange sich beispielsweise ein Laptop in einer erlaubten Zone befindet, wird dies als normaler Zustand erkannt. Wird der "tabuisierte" Laptop aus der Zone entfernt, wird hingegen ein Alarm gegeben oder gemeldet. Das System kann auch unterscheiden, ob der Laptop nicht vielleicht von einer berechtigten, also einer bereits erkannten und bereits identifizierten Person, entfernt worden ist. In diesem Fall würde kein Alarm gegeben oder gemeldet.

Die kognitive Temperatursteuerung ist sinnvoll dann einzusetzen, wenn einer Person die Möglichkeiten abhanden gekommen sind, selbst für ein angenehmes Lebensklima zu sorgen. Es wird vielfach beobachtet, dass demente Personen einfach vergessen, dass sie ein Fenster geöffnet haben, oder dass sie vergessen haben, dass ein Fenster zu schließen ist, wenn die Temperatur im Raum zu weit abgesunken ist. In diesem Fall kann ein in der Nähe des Fensters angebrachter Temperatursensor im Zusammenhang mit der Zonenerkennung, dass nämlich der Fenstergriff eine Offenstellung des Fensters anzeigt, wiederum für einen Alarm sorgen. Dabei hat das System die Möglichkeit, beispielsweise im Raum eine Klingel ertönen zu lassen, so wie eine Telefonklingel, welche die demente Person aus ihrem Nicht-Wahrnehmungszustand erlöst.

Im Folgenden soll die Erfindung anhand der beigefügten Zeichnungen in Einzelheiten beschrieben werden. Dabei zeigt:
- Figur 1: ein stark schematisiertes Blockdiagramm einer Schaltung, mit der ein System gemäß einer Ausführungsform der Erfindung realisiert werden kann;
- Figur 2: ein Gehäuse, in dem ein System gemäß der vorliegenden Erfindung unterzubringen ist;
- Figur 3a: eine bildliche Darstellung, welche die Definition von statischen Zonen erläutert;
- Figur 3b: eine bildliche Darstellung, welche das Konzept einer dynamischen Zone veranschaulicht;
- Figur 4: eine Darstellung, die Konfigurationen des erfindungsgemäßen Systems veranschaulicht; und
- Figur 5: eine Darstellung, mit der die Arbeitsweise des erfindungsgemäßen Systems am Beispiel einer Objektüberwachung veranschaulicht werden soll.

In der nachfolgenden Beschreibung und in den Zeichnungen sind zahlreiche bestimmte Einzelheiten aufgeführt. Es wird jedoch verstanden, dass Ausführungsformen der vorliegenden Erfindung ohne diese bestimmten Einzelheiten in die Praxis umgesetzt werden können. In anderen Fällen sind gut bekannte Schaltungen, Strukturen und Techniken weggelassen, um das Verständnis der Erfindung nicht zu erschweren. Die in den Zeichnungen dargestellten Komponenten sind außerdem nicht notwendigerweise maßstabsgetreu dargestellt. Die dargestellten Ausführungsbeispiele sollen daher als beschreibend und nicht als beschränkend angesehen werden.

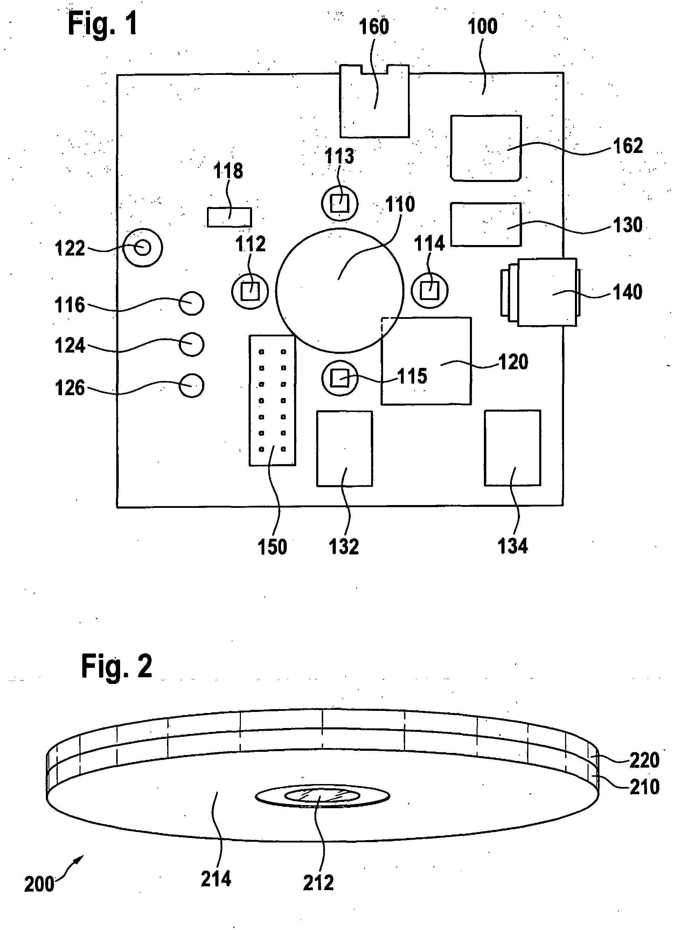

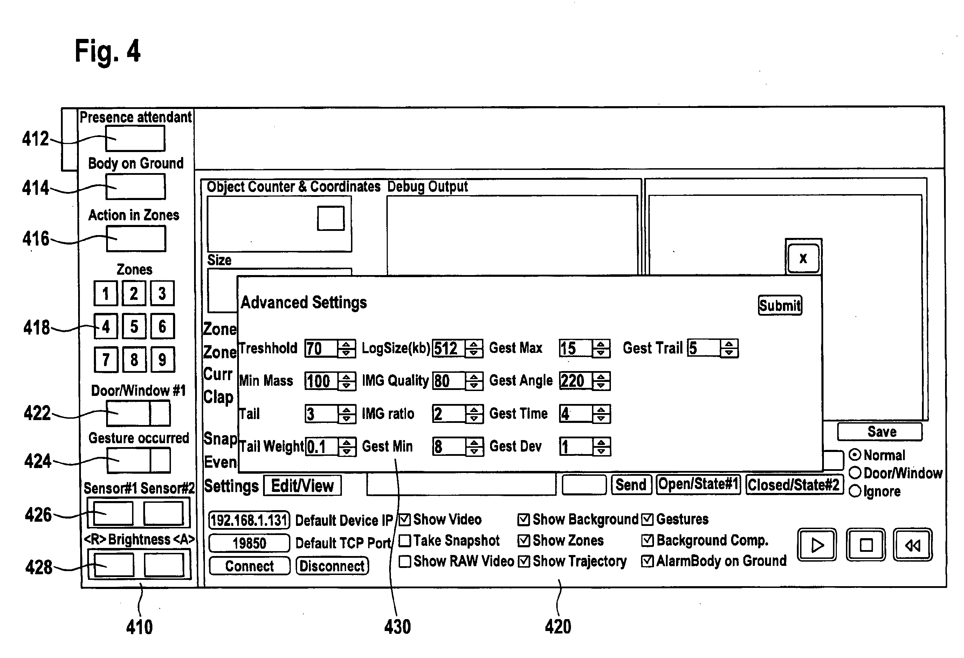

Figur 1 zeigt ein stark schematisiertes Blockdiagramm einer Schaltung, mit der die vorliegende Erfindung in die Praxis umgesetzt werden kann. Die Schaltung ist auf einer Platine 100 aufgebaut, deren Abmessungen etwa 10 cm x 10 cm betragen. Es sind nur die wichtigsten Baugruppen dargestellt, die notwendige Beschaltung mit Widerständen, Kondensatoren usw. ist aus Gründen der Einfachheit der Darstellung weggelassen. Mittig auf der Platine befindet sich eine Kamera 110 mit Fischaugenobjektiv. Die Kamera 110 hat einen Bildwinkel in der Bilddiagonalen, der bis zu 220° betragen kann. Die Kamera 110 hat einen Detektionsbereich bis ins tiefe Infrarot (IR). Zum Ausleuchten sind am Umfangsbereich der Kamera 110 eine oder mehrere Infrarot-LED's 112, 114 angeordnet. Diese dienen außerdem dazu, IR-Sendetelegramme für die Bedienung von IR-Geräten auszusenden. Die Kamera 110 nimmt visuelle Information auf und liefern entsprechende elektrische Signale an eine später noch genauer zu beschreibende Auswertelogik, die durch einen Mikroprozessor 120 realisiert ist. Der Mikroprozessor 120 kann aus Platzgründen zumindest teilweise unter der Kamera 110 angeordnet sein. Die Auswertung der visuellen Information wird durch einen Helligkeitssensor 116 unterstützt, der zum Beispiel der Tag- und Nachterkennung dient und dazu beitragen kann, dass ein Gegenstand nicht als unterschiedlich angesehen wird, wenn er einerseits vom Sonnenlicht bestrahlt wird, andererseits im Schatten liegt. Der Helligkeitssensor 116 wird kalibriert, indem zwei extreme Lichtverhältnisse simuliert werden. Zunächst wird der Raum, in dem das System installiert ist, nahezu komplett abgedunkelt, danach wird eine Sonneneinstrahlung mit Hilfe eines Reflektors simuliert oder der Raum wird mit Sonnenlicht geflutet. Angezeigt wird der unterste Wert Null bei kompletter Dunkelheit. Der Wert MAX liegt bei maximaler Helligkeit vor. In diesem Intervall wird die relative Helligkeit des Raums bestimmt und (Fig. 4; Positionsziffer 428) angezeigt. Eine Vorkalibrierung des Helligkeitssensors 116 ist oftmals möglich, so dass im Raum lediglich eine Nachkalibrierung erfolgen muss. Ein Mikrofon 118 dient zur Aufnahme auditiver Information und kann zur Spracherkennung oder zur Erkennung von Hintergrundgeräuschen eingesetzt werden. Bei diesem Ausführungs-beispiel wird olfaktorische Information lediglich über einen Gassensor 122 erhalten. Es ist aber möglich, weitere Geruchssensoren einzusetzen, wenn dies gewünscht wird. Haptische Information wird über einen Temperatursensor 124 und einen Feuchtesensor 126 erlangt. Die Anordnung der Kamera 110, des Mikrofons 118 und der Sensoren 116, 124, 126 ist weitgehend beliebig und durch die dargestellte Ausführungsform nicht vorgegeben. Als nichtflüchtiger Speicher ist ein Flash-Speicher 130 vorgesehen. Dieser enthält Programme, die von dem Mikroprozessor 120 auszuführen sind, insbesondere auch Lernsoflware für das System, außerdem Datenbanken mit Umgebungsparametern, Nachschlagetabellen für Entscheidungsregeln. Zwei mit doppelter Datengeschwindigkeit arbeitende Speicher mit wahlfreiem Zugriff (DDR-RAMs) 132, 134 werden als flüchtige Speicher eingesetzt und unterstützen die Echtzeit-Verarbeitung der Daten. Für die Kommunikation mit Peripheriegeräten sind eine USB-Schnittstelle 140 und ein JTAG-Adapter 150 vorgesehen. Damit das System mit einem kabelgebundenen Datennetz verwendet werden kann, ist ein Ethernet-Zugang 160 mit zugehörigem Ethernet-Controller 162 vorgesehen. Nicht dargestellt ist ein Funkempfänger zur Kommunikation mit außerhalb der Platine befindlichen Sensoren, beispielsweise zusätzlichen Temperatursensoren. Aus ethischen Gründen und unter Datenschutzaspekten werden alle erlangten Daten im System gehalten und lediglich Meldungen und/oder Alarme abgesetzt.

Figur 2 zeigt eine perspektivische Ansicht eines Gehäuses, in dem eine Platine für ein erfindungsgemäßes System, beispielsweise die bestückte Platine gemäß Figur 1, untergebracht werden kann. Das Gehäuse 200 besteht aus einem tellerartigen Unterteil 210, das beispielsweise aus Kunststoff, wie Propylen, hergestellt sein kann, so wie aus einem Oberteil 220, wobei nach dem Einbau der Platine 100 (Figur 1) und nach der Montage des Gehäuses 200 an der Wand oder an der Decke eines Raumes das Unterteil 210 und das Oberteil 220 von einem Ungefugten nicht mehr zerstörungsfrei voneinander gelöst werden können. Solange das Gehäuse 200 also intakt ist, kann ein unbefugter Eingriff in das System ausgeschlossen werden. Die Platine 100 ist in dem Gehäuse 200 derart angeordnet, dass das Objektiv der Kamera 110 (Figur 1) durch eine entsprechende mittig angeordnete Öffnung 212 im Unterteil 210 schaut. Die Öffnung 212 ist von einem Ring 214 umgegeben, der aus für Infrarot durchlässigem Material besteht, so dass die LED's 112, 114 (Figur 1) Infrarotstrahlung durch diesen Ring 214 hindurch senden können. Bevorzugt sind die Öffnung 212 und der Ring 214 so bemessen, dass bei eingebauter Platine 100 in zusammengesetztem Zustand zwischen dem Objektiv der Kamera 110 und dem Ring 214 ein umlaufender Spalt verbleibt. Durch diesen Spalt können Schall, Gase, Dämpfe usw. in das Innere des Gehäuses 200 eintreten, so dass die entsprechenden Sensoren, z.B. der Gassensor 122 (Figur 1) angesprochen werden können. Als Alternative können das Unterteil 210 des Gehäuses 200 und der Ring 214 so gestaltet werden, dass am Außenumfang des Rings 214 ein Spalt gebildet wird. Auch eine Kombination dieser beiden Möglichkeiten ist denkbar. Das Gehäuse 200 weist eine geringe Bauhöhe auf, beispielsweise 1 cm bis 2 cm, so dass es sich in einen Raum gut integrieren lässt. So kann das Gehäuse beispielsweise als ein ästhetisch anmutendes Gestaltungsmerkmal an der Decke eines Wohnzimmers oder Arbeitszimmers angebracht werden.

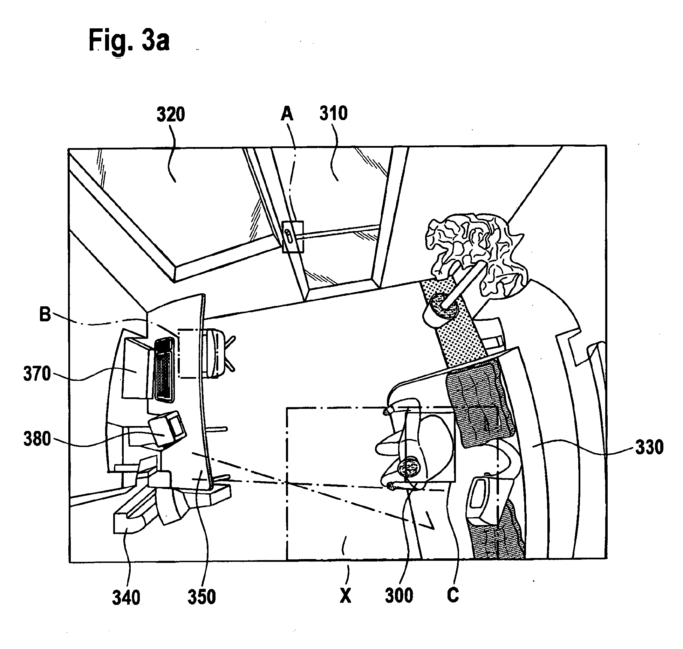

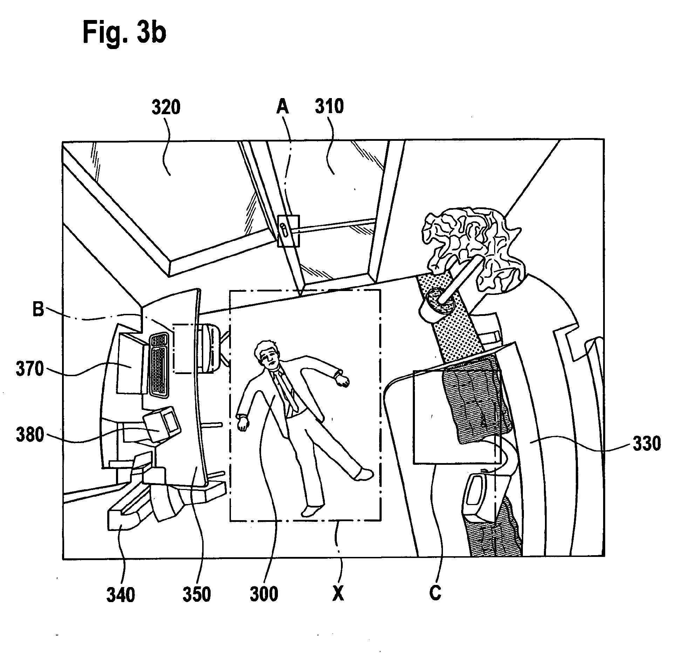

Figur 3a zeigt, wie erfindungsgemäß eine Aufteilung eines Raumes in Zonen erfolgen kann. Beispielhaft dargestellt ist ein Wohn-/Arbeitszimmer aus der Perspektive einer Kamera, die sich, beispielsweise in das Gehäuse der Figur 2 eingebaut, an der Decke des Raumes befindet. Die Kamera bildet den Raum ab, so dass eine Karte des Raumes, wie in der Figur 3a gezeigt, erstellt werden kann, wobei in dieser Karte nun, wie im Folgenden erläutert wird, zunächst eine Anzahl statischer Zonen definiert wird. Der dargestellte Raum weist eine Terrassentür 310 sowie ein danebenliegendes Fenster 320 auf. Im unteren rechten Kartenbereich befindet sich ein Sofa 330, auf dem in der gezeigten Szene eine Person 300 sitzt. Gegenüber dem Sofa 330 befindet sich, zwischen einem Bücherregal 340 und dem Fenster 320, ein Arbeitstisch 350 mit davor angeordnetem Stuhl 360. Auf dem Arbeitstisch 350 befindet sich ein Anzeigebildschirm mit Tastatur 370 sowie ein Laptop 380. Auf der Karte werden nun Zonen definiert, deren Position, Anzahl und Größe sich nach dem gewünschten Anwendungszweck richtet. Eine erste Zone A ist an der Terrassentür 310 definiert, und zwar den Türgriff umgebend. Je nach Stellung des Türgriffes lässt sich ableiten, ob die Terrassentür geschlossen, geöffnet oder auf Kipp gestellt ist. Zusätzlich kann dem System vorab visuelle Information darüber gegeben werden, ob die Türrahmen und die Zarge beispielsweise in der Kippstellung und in der Schließstellung zueinander stehen. Das System kann also nur mit Information aus der Zone A - und nicht etwas an der gesamten Tür oder gar aus dem gesamten Raum - eine Aussage über den Zustand der Tür treffen. Eine zweite Zone B umfasst den Bereich der Arbeitsplatte des Arbeitstisches 350 vor dem Anzeigebildschirm und der Tastatur 370 sowie einen Teil des Stellbereiches des Arbeitsstuhles 360. In dieser Zone B sind Aktivitäten eines Benutzers zu erwarten. Wenn sich beispielsweise die Person 300 auf den Arbeitsstuhl 360 setzen würde, würde eine Veränderung in der Zone B vom System detektiert. Beispielsweise könnte das System, nachdem es erkannt hat, dass die Person 300 jetzt am Arbeitstisch 350 sitzt, eine Schreibtischlampe oder auch den mit dem Anzeigebildschirm und der Tastatur verbundenen Computer anschalten. Eine dritte Zone C deckt einen Teil der Sitzfläche des Sofas 330 ab. Bei der dargestellten Szene sitzt die Person 300 auf dem Sofa 330 und löst damit eine Änderung in der Zone C aus, wenn sie sich bewegt. Daraus kann das erfindungsgemäße System beispielsweise schließen, dass die Person 300 nicht schläft, und es kann die Zimmerbeleuchtung und Zimmertemperatur beispielsweise nach voreingestellten Wünschen regeln. Die Zonen A, B und C sind statische Zonen, die im Regelfall nicht ständig geändert werden, sondern allenfalls neu zu konfigurieren sind, wenn sich die Bedürfnisse des Benutzers oder die räumliche Situation ändern. Eine dynamische Zone X definiert sich das erfindungsgemäße System selbst aus Umriss- und Bewegungsdaten der Person 300. Dies ist genauer in Figur 3b veranschaulicht. Hier ist eine rechteckige dynamische Zone X durch das System so eingerichtet, dass die Umrisslinie der Person 300 darin enthalten ist. Bei einer dynamischen Zone X kann es kritisch sein, wenn über einen kürzeren oder längeren Zeitraum keine Änderungen detektiert werden, ihr Zustand also konstant bleibt. Beispielsweise kann dies der Fall sein, wenn die Person 300 stürzt. Wenn sie länger als etwa 4 bis 10 Sekunden regungslos liegenbleibt, detektiert das System einen anomalen Zustand und trifft die Entscheidung, als Aktion einen Alarm auszugeben. Die dynamische Zone X ist somit eine Zone, die ständig überwacht und auf vordefinierte Zustände und Zustandsänderungen überprüft wird.

Das erfindungsgemäße System identifiziert Personen oder Gegenstände durch den Umriss, der in der Figur 3 durch eine punktierte Linie dargestellt ist. Da im Regelfall nur eine beschränkte Anzahl von Personen sich in dem Raum aufhalten wird, kann der Umriss nicht nur zum Erkennen, sondern auch zum Identifizieren der Person benutzt werden. Gleiches gilt für Gegenstände. Wenn beispielsweise der aufgeklappte Laptop 380 statt auf dem Arbeitstisch 350 auf dem Sofa 330 und dort in der Zone C abgestellt würde, dann würde das System dieses erkennen und bestimmte Aktionen unterlassen, die es bewirken würde, wenn auf dem Sofa 330 eine Person 300 Platz genommen hätte.

Das erfindungsgemäße System kann anhand von Gesten einer Person, für die das System eine dynamische Zone X definiert hat, erkennen, welche Funktion gewünscht ist. Dazu muss dem System vorab erklärt werden, wann eine Bewegung als Geste zu werten ist und wann nicht. Rein intuitiv würde eine Person, wenn sie auf einen Gegenstand zeigt, den Arm lang in diese Richtung ausstrecken, so dass ein möglicher Ansatz zur Definition einer Geste wäre, den Winkel zwischen Unterarm und Oberarm zu bestimmen, wobei diese Stellung über eine vorab festgelegte Zeitdauer, beispielsweise wenige Sekunden, gehalten werden muss. Das erfindungsgemäße System kann nun, wenn eine Bewegung als Geste identifiziert ist, neben den Positionskoordinaten der Person, die diese Geste durchführt, auch die Richtung angeben, in die sie zeigt. Diese Richtungsangabe wird zweckmäßigerweise in Bezug auf ein raumfestes Koordinatensystem bestimmt. Die Definition einer dynamischen Zone X für eine Person verhindert dabei, dass unberechtigte Personen Gestiken durchführen, die zu einer nicht gewünschten Aktion fiühren. Auch Fehler, die durch wehende Fenstervorhänge oder dergleichen hervorgerufen werden könnten, werden ausgeschaltet.

Die Bewegungserkennung, die einerseits für die Definition einer dynamischen Zone, andererseits für die Gestikerkennung erforderlich ist, wird durch die Kombination zweier Basistechniken erzielt, nämlich der differentiellen Analyse des Bildes sowie der in anderem Zusammenhang schon angesprochenen Hintergrundkompensation. Die differentielle Analyse des Bildes besteht aus einem Vergleich von aufeinanderfolgenden Frames auf einer Basis von Pixel zu Pixel. In einem definierten Grenzbereich bzw. Intervall werden die Änderungen der Leuchtstärke und der Farbe einzelner Pixel erkannt, um die Bestandteile des sich bewegenden Objekts zu bestimmen. Ein Pixel ist als Bestandteil des sich bewegenden Objekts markiert, wenn die Änderung seiner Parameter über einen definierten, konfigurierbaren Grenzbereich hinausgeht. Die Hintergrundkompensation basiert auf der Aufrechterhaltung eines Hintergrundbildes über einen längeren Zeitraum, bestehend aus den Pixeln, die über mehrere aufeinanderfolgende Frames nicht wesentlich verändert wurden. Die zeitliche Abfolge der Parameterwerte der einzelnen Pixel wird untersucht und unter bestimmten Stabilitätskriterien, die ebenfalls konfigurierbar sind, als Teil des Hintergrunds klassifiziert. Daraufhin wird jedes neue Frame auf das aktuelle Hintergrundbild gesetzt und mit diesem verglichen. Somit gelingt es, bewegte Objekte vor einem wahrscheinlich kontinuierlich stabilen Hintergrund zu erkenne. Dabei passt sich das Hintergrundbild automatisch auf externe Einflüsse, gegeben beispielsweise durch die Tageszeit, an. Im Gegensatz zur rein differentiellen Bewegungserkennung, die auf einer deutlich kürzeren zeitlichen Skala arbeitet, wird durch eine derartige gewichtete Mittelwertbildung mehrerer vorangegangener Frames mit konfigurierbarer Gewichtung und Sequenzlänge eine Verbesserung der Objekt- und Mustererkennung erreicht. (Tail) Die Kombination beider Techniken ermöglicht eine höhere Zuverlässigkeit in Bezug auf sich bewegende Objekte, die eine unterschiedliche Geschwindigkeit oder Position haben.

Nach Erkennung der sich verändernden Pixel, die - wahrscheinlich - zum untersuchten Objekt gehören, werden Cluster identifiziert. Cluster sind vereinzelte und benachbarte Pixelgruppen, die zum sich bewegenden Objekt gehören und deren Parameter sich nach den oben beschriebenen Methoden verändert haben. Dies wird unter anderem durch den Einsatz des Hoshen-Kopelman Algorithmus oder anderen der Perkolationstheorie zugrundeliegenden Algorithmen im linearen Zeitbereich erreicht, ohne dass fehleranfällige rekursive Funktionen notwendig wären.

Die identifizierten Cluster werden dann durch die K-Means Clustering-Technik analysiert, die letztendlich dem individuellen, sich bewegenden Objekt einen definierten Schwerpunkt und ein geschlossenes zusammenhängendes Gebilde zuordnet.

Dabei wird abgesehen von Querbewegungen der Objekte auch eine Tiefenerkennung der Objekte angewandt. Die Bewegung des Objekts wird analysiert, ohne dass es zu nennenswerten seitlichen Bewegungen des Objektes kommen muss. Im Zusammenspiel mit den vorher geschilderten Techniken zur Bewegungserkennung ist eine Erkennung auch möglich, wenn sich das Objekt nicht nur im zweidimensionalen X-Y-Koordinatensystem bewegt, sondern auch in der dritten Dimension Z. Aus der Tiefenbewegung des Objektes ergibt sich eine Veränderung, die beispielsweise eine Verkleinerung des Objektes aus der wachsenden Entfernung zur Kamera hin bedeutet. Die kurz- und längerfristige Analyse dieses Änderungsvorgangs ermöglicht eine präzisere Unterscheidung mehrerer Objekte im Raum im Hinblick auf deren Größe. Auch wird eine präzisere Gestikerkennung ermöglicht, so dass eine direkte Ausrichtung zur Kamera nicht notwendig ist. Die Gestiken können auch bei kleineren Abweichungen zur direkten Ausrichtung zur Kamera hin erkannt werden, falls vorher eine hinreichend lange Analyse der Bewegungen des Objektes erfolgt ist.

Das erfindungsgemäße System muss vorab mit der erforderlichen Information versorgt werden. Dazu dient der Konfigurationsvorgang, der im Folgenden im Zusammenhang mit der Figur 4 beschrieben werden soll. Dabei wird ausgenutzt, dass das erfindungsgemäße System als lernendes System gestaltet ist. Die Figur 4 zeigt einen Konfigurationsbildschirm mit einem Statusbereich 410 und einem Dokumentationsbereich 420. Im Dokumentationsbereich 420 ist ein Bildschirmfenster 430 geöffnet, an dem diverse Einstellungen vorgenommen werden können.

Im Statusbereich 410 erfolgt die Anzeige von Zuständen mit Hilfe von Feldern, die ihre Farbe ändern. Natürlich können die Zustände auch auf andere Weise sichtbar gemacht werden, dies liegt im Ermessen des Fachmanns.

Der Statusbereich 410 weist zunächst ein Fenster 412 auf, in dem angezeigt wird, ob sich eine Person in dem Raum befindet (*Presence attendant*). Solange keine Person in dem Raum detektiert worden ist, bleibt das Feld 412 schwarz. Sobald eine Person anwesend ist, leuchtet das Feld 412 grün auf.

Die Personen und/oder Gegenstände, die sich im Sichtbereich der Kamera aufhalten, werden nach Größe bzw. nach Masse unterschieden, und es werden entsprechend unterschiedliche Alarme signalisiert. Für den Fall, dass eine erwachsene Person dieselbe Größe bzw. Masse wie ein Kind hat, wird nicht zwischen Alarm für einen Erwachsenen und Alarm für ein Kind unterschieden. Eine sinnvolle und alltagsgerechte Aufteilung der Objekte nach Größe/Masse ist hier erforderlich.

Ein Feld 414 zeigt an, ob eine mögliche Notsituation entstanden ist (*body on ground*). Solange dies nicht der Fall ist, bleibt das Feld 414 neutral grau, falls eine Person am Boden liegend festgestellt wird, und das System annehmen muss, das diese gestürzt ist und sich nicht ohne fremde Hilfe wieder erheben kann, leuchtet das Feld rot.

Wenn ein Notruf abgesetzt werden soll, wenn sich ein Mensch reglos im Raum befindet, muss zunächst festgestellt werden, ob ein normaler Zustand oder ein alarmierender Zustand vorliegt. Dazu muss für diese Position vorab das Bett bzw. eine andere Liegeeinrichtung so genau wie möglich durch eine Zone markiert werden. Für das Lernverhalten betritt nun eine Person den Raum und bewegt sich beliebig oft und lange im Raum. Ein normaler Zustand bestände darin, dass die Person zum Bett geht, sich hinlegt und nach einer gewissen Zeit t nicht mehr bewegt. Es soll vom System lediglich die Position automatisch protokolliert werden.

Bei einem alarmierenden Zustand bewegt sich die Person beliebig im Sichtbereich der Kamera und dann hört die Bewegung der Person plötzlich auf - beispielsweise wegen eines Unfalls oder einer Ohnmacht, und die Person bleibt regungslos außerhalb der markierten Liegezone. In diesem Fall wird nach einer Zeit t₀ ein Alarm ausgelöst.

Ein Feld 416 gibt an, ob in einer der definierten Zonen (z.B. A, B, C, ... X in Figur 3a bzw. Figur 3b) Änderungen feststellbar sind (*action in zones*). Ist dies nicht der Fall, bleibt das Feld 416 neutral grau, ansonsten leuchtet es grün.

Beim vorliegenden Konfigurationsbildschirm besteht die Möglichkeit, neun Zonen (*zones*) zu definieren, die mit 1, 2, 3, ..., 9 durchnumeriert sind. Die Anzeigefelder für die Zonen sind in einer matrixartigen Anordnung 418 zusammengefasst.

Ein Feld 422 informiert über den Zustand der Tür bzw. des Fensters. Solange eine Tür bzw. ein Fenster geschlossen sind, bleibt das Feld 422 neutral grau, wenn eines oder beide offen sind, leuchtet das Feldsignal rot.

Die Fenster und Türen, die von Interesse sind, müssen sich im Sichtbereich der Kamera befinden. Es ist erforderlich, dass das System einen Lernprozess durchläuft, damit es den Unterschied zwischen einem geschlossenen Fenster und einer geschlossenen Tür und einem geöffneten Fenster und einer geöffneten Tür kennt. Dem System wird dazu einmal das Fenster bzw. die Tür im geschlossenen Zustand gezeigt und einmal in geöffnetem Zustand. Darüber hinaus kann nach entsprechender Kalibrierung auch zwischen halbgeöffneten Zuständen unterschieden werden, wenn dies gewünscht wird.

Die Person, deren Gesten erkannt werden sollen, muss sich im Sichtbereich der Kamera aufhalten und zu dieser hin gedreht sein. In Abhängigkeit des Abstandes der Person zur Kamera muss die Person bei größerer Entfernung zu einer größeren Gestik ausholen als bei kleinerer Entfernung. Die Kalibrierung wird später genau mit Bezug auf das Bildschirmfenster 430 beschrieben.

Ein Feld 424 zeigt an, ob die Person eine Geste ausgeführt hat. Solange dies nicht geschehen ist, bleibt das Feld 424 neutral grau, ansonsten leuchtet es grün. Sowohl das Feld 422 als auch das Feld 424 können ein (schwarz hinterlegtes) Zählfeld enthalten, indem die entsprechende Anzahl der Ereignisse nachgehalten wird. Zwei Felder 426 sind für Sensoren vorgesehen (*sensor*#*1, sensor*#*2*), beispielsweise für den Gassensor und den Temperatursensor, wobei jeweils Farbänderungen erfolgen, wenn der Gassensor ein Gas detektiert oder der Temperatursensor feststellt, dass bestimmte Temperaturwerte über- oder unterschritten werden. Schließlich sind zwei Felder 428 vorgesehen, die Angaben über die Helligkeit (*brightness*) geben, welche von dem Helligkeitssensor (116 in Figur 1) geliefert werden.

Wird vom System ein Geräusch registriert, beispielsweise über ein Mikrofon (118 in Figur 1), so wird der Pegel zunächst protokolliert. Kombiniert wird das Mikrofon zweckmäßigerweise mit dem visuell arbeitenden Präsenzmelder. Sollte sich kein Objekt im Bildbereich der Kamera bewegen, das System jedoch ein ausreichend starkes Geräusch aufnehmen, so wird die Anwesenheit mindestens einer Person angezeigt. Eine Unterscheidung der Anzahl der Personen erfolgt in diesem Fall nicht. Zusätzlich kann eingestellt werden, welche Alarme, Meldungen oder Funktionen ausgelöst werden sollen, falls eine Person in die Hände klatscht.

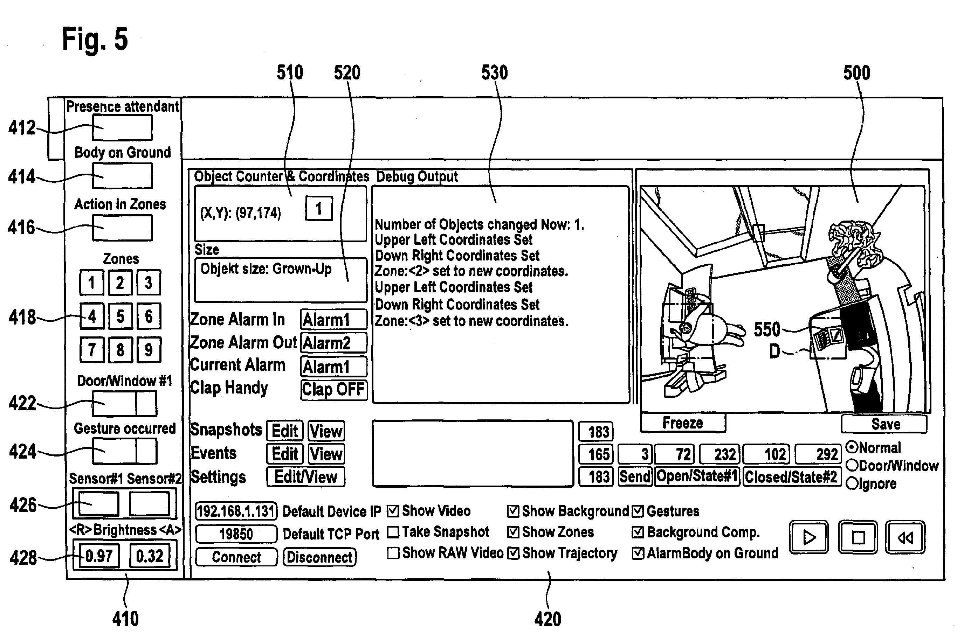

Der Dokumentationsbereich 420 wird später im Zusammenhang mit Figur 5 beschrieben.

Das Bildschirmfenster 430 zeigt ein Beispiel für die Programmierung des lernfähigen, erfindungsgemäßen Systems für die Gestikerkennung, bei der änderbare Parameter (*Advanced Settings*) eingestellt werden, die die Bildverarbeitung optimieren. So werden zunächst ein Schwellenwert (*Threshold*), der festlegt, ob überhaupt eine Bewegung erkannt wird, und eine minimale Masse (*Min Mass*), die als Cluster erkannt wird, festgelegt. Die zuvor schon angesprochene Auswertung über die Historie der Frames nach Anzahl (*Tail*) und Gewichtung (*Tail Weight*) bezieht Vergangenheitsbilder mit ein. Die Bilder werden für die Speicherung im Flash-Speicher (130 in Figur 1) komprimiert (*Log Size (kb), IMG Quality, IMG ratio*). Eine Geste wird durch minimal und maximal erforderliche Länge (*Gest Min, Gest Max*) eines als Arm identifizierten Clusters, durch einen Winkel (*Gest Angle*), eine Dauer (*Gest Time*), erlaubte Abweichungen (*Gest Dev*) und Bezug auf eine Anzahl von vergangenen Gesten (*Gest Trail*) festgelegt. Durch "Submit" werden die Parameter im System gespeichert.

Figur 5 zeigt einen Bildschirm nach der Konfiguration im Einsatz. Der Statusbereich 410 wurde bereits im Zusammenhang mit Figur 4 erläutert. Das System hat festgestellt, dass sich Personen im Raum befinden (Feld 412), dass sich keine Person am Boden befindet (Feld 414) und dass in den Zonen Änderungen geschehen (Feld 416), und zwar in den Zonen 2 und 3 (Feld 418). Tür und Fenster sind geschlossen (Feld 422), es wurde keine Gestik erkannt (Feld 424), die Sensoren haben keine anomale Situation erfasst (Feld 426), die Helligkeit ist entsprechend abgeglichen (Feld 428).

Der Dokumentationsbereich 420 weist ein Farbdisplay 500 auf, das im Wesentlichen nur bei der Konfiguration des Systems eingesetzt und anschließend im Regelfall deaktiviert wird. Für das erfindungsgemäße System ist es aus Datenschutzgründen lediglich geplant, dass bestimmte Zustände einen Alarm oder eine Meldung auslösen, dass aber normalerweise kein Bildmaterial ausgegeben wird, so dass eine wie auch immer geartete Überwachungskamera nicht funktioniert und die Privatsphäre unbedingt gewahrt bleibt. Es mag aber besondere Situationen geben, in denen es nicht nur wünschenswert, sondern sogar notwendig ist, eine Bildschirmfunktion zu aktivieren. Dies ist beispielsweise der Fall, wenn gebrechliche, insbesondere demente Personen mit dem erfindungsgemäßen System unterstützt werden sollen. Zum Beispiel kann es sehr sinnvoll sein, einen Schnappschuss aufzunehmen, wenn eine Person reglos am Boden liegend detektiert wird.

Das System hat nun festgestellt, dass sich eine Person in dem Raum befindet, was im Feld 510 mittels der aktuellen Positionskoordinaten für die Person und einer Anzahl im Zählfeld festgehalten wird. Gemäß 520 ist die Person als eine erwachsene Person identifiziert worden. Aus dem Protokoll im Feld 530 geht der Ablauf, seit die Person den Raum betreten hat, hervor. Die Koordinaten der Person sowie der in dem Raum definierten Zonen wird festgehalten.

Anhand von Figur 5 soll außerdem erläutert werden, wie mit Hilfe des erfindungsgemäßen Systems ein Objektschutz durchgerührt werden kann. Im Bildbereich des Farbdisplays 500 ist zu erkennen, dass um das zu schützende Objekt, in diesem Fall ein Laptop, der sich auf einem Sofa befindet, eine statische Zone D definiert ist. Das erfindungsgemäße System erkennt sowohl den Laptop, als auch, dass er sich in der Zone D befindet. Für diese Zone D wird nun ein Alarm gesetzt, der beispielsweise darin besteht, dass eine entsprechende SMS auf das Handy eines Berechtigten geschickt wird. Solange der Berechtigte keine diesbezügliche SMS erhält, weiß er, dass der Laptop nach wie vor an seinem Platz ist. Wird im Fall eines Einbruchs der Laptop vom Sofa genommen, so detektiert das System eine Änderung in der Zone D, der Alarm wird ausgelöst und die SMS verschickt, so dass der Berechtigte sogleich entsprechende Schritte einleiten und etwa die Polizei alarmieren kann.

Für einen solchen Fall kann auch die Bildschirmfunktion aktiviert werden. Sobald der Alarm ausgelöst wird, nimmt das System einen Schnappschuss der Szene auf, der später weiterverwendet werden kann.

Durch das Prinzip der Zonen wird die Rechenarbeit für das System enorm verringert. Insbesondere die Gestiksteuerung wird dadurch erst in sinnvoller Weise möglich. Um eine Änderung als Gestik auszumachen, braucht das System lediglich die dynamische Zone X, die für eine Person definiert wird, zu betrachten. Die mit der Gestik zu steuernden Geräte oder Schalter werden vorab definiert und dem System mitgeteilt. Da es nicht mehr erforderlich ist, einen Schalter, Taster oder Touchscreen direkt zu berühren, wird es auch für ältere und gebrechliche Personen kein Problem mehr darstellen, Geräte zu bedienen und somit eigenständig und komfortabel zu agieren.

Auch die Steuerung von IR-Geräten wird möglich, wenn der Benutzer eine statische Zone - zum Beispiel die Couch vor einem Fernseher - erstellt und als Option das Bedienen des IR-Gerätes wählt. Betritt nun eine Person diese Zone, so wird nach einer Zeit t₁ das IR-Gerät angeschaltet. Verlässt die Person die Zone, wird das Gerät nach einer Zeit t₂ ausgeschaltet. Eine IR-Meldung kann dabei direkt von in dem System vorhandenen IR-LED's ausgegeben werden.

Die in der vorstehenden Beschreibung, in der Zeichnung sowie in den Ansprüchen offenbarten Merkmale der Erfindung können sowohl einzeln als auch in beliebiger Kombination für die Verwirklichung der Erfindung wesentlich sein.

## Patentansprüche

1. System zum Verarbeiten von visueller, auditiver, olfaktorischer und/oder haptischer Information für das kognitive Bestimmen wenigstens eines Zustandes mindestens eines Raumes und/oder mindestens einer Person und/oder mindestens eines Gegenstandes und zum Finden und Umsetzen einer Entscheidung abhängig von dem wenigstens einen erkannten Zustand, das aufweist:
- ein erstes Erkennungsmodul (110), das zum Feststellen mindestens der Anwesenheit und gegebenenfalls zum Identifizieren von Personen und/oder Gegenständen ausgelegt ist und ein entsprechendes elektrisches Signal ausgibt;
- einen Satz Sensoren (116, 118, 122, 124), wobei der Satz Sensoren dazu ausgelegt ist, visuelle, auditive, olfaktorische und/oder haptische Information quantitativ oder qualitativ aufzunehmen und entsprechende elektrische Signale auszugeben; und
- eine Auswertelogik (12) zum Verarbeiten der zumindest vom ersten Erkennungsmodul und von dem Satz Sensoren (116, 118, 122, 124) ausgegebenen Signale;
wobei für den mindestens einen Raum eine zwei- oder dreidimensionale Karte zur Verfügung gestellt ist, welche eine oder mehrere statische Zonen (A, B, C) aufweist, wobei jede statische Zone (A, B, C) ein Gebiet des Raumes kennzeichnet, aus dem visuelle, auditive, olfaktorische und/oder haptische Information über den Zustand des Raumes, einer Person und/oder eines Gegenstandes (310, 330, 350) zu erlangen ist,
**dadurch gekennzeichnet, dass**
eine Einrichtung vorgesehen ist, welche, sobald das erste Erkennungsmodul (110) die Anwesenheit einer oder mehrerer Personen (300) in dem Raum signalisiert, für zumindest eine dieser Personen eine dynamische Zone (X) aus Umrissdaten derselben definiert, aus welcher Bewegungsinformation von der oder über die Person (300) zu erlangen ist,
dass ein zweites Erkennungsmodul vorgesehen ist, das zum Detektieren von Bewegungen einer Person ausgelegt ist, wobei das zweite Erkennungsmodul Information lediglich aus einer dynamischen Zone (X) erhält, und dass
die Auswertelogik (120) einen Zustand des Raumes und/oder einer Person (300) und/oder eines Gegenstandes aus den verarbeiteten Signalen erkennt, wobei zum Erkennen des Zustandes an visueller Information nur solche verwendet wird, die aus einer der Zonen herrührt, wobei, wenn Umrissänderungen der dynamischen Zone (X) erkannt werden, die Auswertelogik (120) auf Bewegungen der zugeordneten Person rückschließt, und die Auswertelogik (120) eine Entscheidung trifft, basierend auf dem erkannten Zustand, ob eine Aktion einzuleiten ist oder nicht.

2. System nach Anspruch 1, **dadurch gekennzeichnet, dass** das erste Erkennungsmodul den Umriss einer Person oder eines Gegenstandes feststellt und deren/dessen Identität anhand des Umrisses festlegt.

3. System nach Anspruch 1 **dadurch gekennzeichnet, dass** das erste und/oder das zweite Erkennungsmodul Teil der Auswertelogik (120) sind.

4. System nach Anspruch 1, **dadurch gekennzeichnet, dass** die Aktion eine Meldung an einen externen Empfänger ist.

5. System nach Anspruch 1, **dadurch gekennzeichnet, dass** zum Aufnehmen visueller Information aus allen Zonen eine einzige Kamera (110) vorgesehen ist.

6. Verfahren zum Verarbeiten von visueller, auditiver, olfaktorischer und/oder haptischer Information für das kognitive Bestimmen wenigstens eines Zustandes mindestens eines Raumes und/oder mindestens einer Person und/oder mindestens eines Gegenstandes und zum Finden und Umsetzen einer Entscheidung abhängig von dem wenigstens einen erkannten Zustand, mit den Schritten:
- Feststellen mindestens der Anwesenheit und gegebenenfalls Identifizieren von Personen und/oder Gegenständen und Ausgeben eines entsprechenden Signals;
- quantitatives oder qualitatives Aufnehmen visueller, auditiver, olfaktorischer und/oder haptischer Information und Ausgeben entsprechender elektrischer Signale; und
- Verarbeiten der ausgegebenen Signale;
- Definieren einer oder mehrerer statischer Zonen (A, B, C) für einen Raum, wobei jede Zone ein Gebiet des Raumes kennzeichnet, aus dem visuelle, auditive, olfaktorische und/oder haptische Information über den Zustand des Raumes, einer Person und/oder eines Gegenstandes zu erlangen ist;
**gekennzeichnet durch**
- sobald die Anwesenheit einer oder mehrerer Personen (300) in dem Raum festgestellt ist, Definieren einer dynamischen Zone (X) für zumindest eine dieser Personen aus Umrissdaten derselben, aus welcher Bewegungsinformation zu erlangen ist;
- Erkennen eines Zustandes des Raumes und/oder einer Person und/oder eines Gegenstandes aus den verarbeiteten Signalen, wobei zum Erkennen des Zustandes an visueller Information nur solche verwendet wird, die aus einer der Zonen (A, B, C, X) herrührt;
- Detektieren von Bewegungen einer Person (300), wobei Information lediglich aus der dynamischen Zone (X), die dieser Person (300) zugeordnet ist, verwendet wird, und
- Entscheiden, basierend auf dem erkannten Zustand, ob eine Aktion eingeleitet wird oder nicht.

7. Verfahren nach Anspruch 6, **gekennzeichnet durch** ,
- Festlegen eines Satzes von Bedingungen;
- Definieren einer oder mehrerer temporärer statischer Zonen (A, B, C) für jede Bedingung aus dem Satz von Bedingungen;
- Prüfen, ob mindestens eine Bedingung eingetreten ist; und
- Aktivieren der temporären, statischen Zone(n) (A, B, C), die für die mindestens eine Bedingung definiert ist; und
- Erlagen von visueller Information aus der/den temporären, statischen Zone(n).

8. Verfahren nach Anspruch 6, **dadurch gekennzeichnet, dass** der Umriss einer Person (300) oder eines Gegenstandes (380) festgestellt und deren/dessen Identität anhand des Umrisses festgelegt wird.

9. Verfahren nach Anspruch 6, **dadurch gekennzeichnet, dass** die Aktion eine Meldung an einen externen Empfänger ist.

## Claims

1. A system for processing visual, auditory, olfactory, and/or haptic information for the cognitive determination of at least one state of at least one room and/or of at least one person and/or of at least one object, and for arriving at and implementing a decision as a function of the at least one recognized state, which system comprises:
- a first recognition module (110) that is designed at least to ascertain the presence of, and optionally to identify, persons and/or objects, and that outputs a corresponding electrical signal;
- a set of sensors (116, 118, 122, 124), the set of sensors being designed to quantitatively or qualitatively acquire visual, auditory, olfactory, and/or haptic information and to output corresponding electrical signals; and
- an evaluation logic system (120) for processing the signals outputted at least from the first recognition module and from the set of sensors (116, 118, 122, 124),
wherein, for the at least one room, a two- or three-dimensional map that comprises one or more static zones (A, B, C) being made available, each static zone (A, B, C) characterizing a region of the room from which visual, auditory, olfactory, and/or haptic information regarding the state of the room, of a person, and/or of an object(310, 330, 350) is to be obtained;
**characterized in that**
a device is provided which, as soon as the first recognition module (110) signals the presence of one or more persons (300) in the room, defines for at least one of said persons a dynamic zone (X) from contour data thereof, from which motion information from or about the person (300) is to be obtained;
a second recognition module is provided which is designed to detect motions of a person, the second recognition module obtaining information only from a dynamic zone (X); and
the evaluation logic system (120) recognizes from the processed signals a state of the room and/or of a person (300) and/or of an object, only that visual information which derives from one of the zones being used to recognize the state, the evaluation logic system (12) inferring motions of the associated person when contour changes of the dynamic zone (X) are recognized, and the evaluation logic system (120) making a decision, based on the recognized state, as to whether or not an action is to be initiated.

2. The system according to Claim 1, **characterized in that** the first recognition module ascertains the contour of a person or of an object, and determines his/her/its identity on the basis of the contour.

3. The system according to Claim 1, **characterized in that** the first and/or the second recognition module are part of the evaluation logic system (120).

4. The system according to Claim 1, **characterized in that** the action is a message to an external receiver.

5. The system according to Claim 1, **characterized in that** a single camera (110) is provided for acquiring visual information from all zones.

6. A method for processing visual, auditory, olfactory, and/or haptic information for the cognitive determination of at least one state of at least one room and/or of at least one person and/or of at least one object, and for arriving at and implementing a decision as a function of the at least one recognized state, having the steps of:
- at least ascertaining the presence of, and optionally identifying, persons and/or objects, and outputting a corresponding signal;
- quantitatively or qualitatively acquiring visual, auditory, olfactory, and/or haptic information and outputting corresponding electrical signals; and
- processing the outputted signals,
- defining one or more static zones (A, B, C) for a room, each zone characterizing a region of the room from which visual, auditory, olfactory, and/or haptic information is to be obtained regarding the state of the room, of a person, and/or of an object;
**characterized by**
- as soon as the presence of one or more persons (300) in the room is ascertained, defining a dynamic zone (X) for at least one of said persons, from which motion information is to be obtained;
- recognizing from the processed signals a state of the room and/or of a person and/or of an object, only that visual information which derives from one of the zones (A, B, C, X) being used to recognize the state;
- detecting motions of a person (300), information only from the dynamic zone (X) associated with that person (300) being used; and
- deciding, based on the recognized state, as to whether or not an action is to be initiated.

7. The method according to Claim 6, **characterized by**
- specifying a set of conditions;
- defining one or more temporary static zones (A, B, C) for each condition from the set of conditions;
- checking as to whether at least one condition has occurred; and
- activating the temporary static zone(s) (A, B, C) that is defined for the at least one condition; and
- obtaining visual information from the temporary static zone(s).

8. The method according to Claim 6, wherein the contour of a person (300) or of an object (380) is ascertained, and his/her/its identity is determined on the basis of the contour.

9. The method according to Claim 6, wherein the action is a message to an external receiver.

## Revendications

1. Système de traitement d'informations visuelles, auditives, olfactives et/ou haptiques pour la détermination cognitive d'au moins un état d'au moins un espace et/ou d'au moins une personne et/ou d'au moins un objet et pour trouver et prendre une décision en fonction de l'au moins un état détecté, qui comprend :
- un premier module de détection (110) conçu pour la détermination de la présence et, le cas échéant, pour l'identification de personnes et/ou d'objets, qui émet un signal électrique correspondant ;
- un ensemble de capteurs (116, 118, 122, 124), cet ensemble de capteurs étant conçu pour enregistrer, de manière quantitative ou qualitative, des informations visuelles, auditives, olfactives et/ou haptiques ; et
- une logique d'analyse (12) pour le traitement des signaux émis au moins par le premier module de détection et de l'ensemble de capteurs (116, 118, 122, 124) ;
Une carte bi- ou tridimensionnelle étant mis à disposition pour l'au moins un espace, qui comprend une ou plusieurs zones statiques (A, B, C), chaque zone statique (A, B, C) identifiant une partie de l'espace, à partir de laquelle des informations visuelles, auditives, olfactives et/ou haptiques concernant l'état de l'espace, d'une personne et/ou d'un objet (310, 330, 350) doivent être obtenues,
**caractérisé en ce que**
un dispositif est prévu, qui, dès que le premier module de détection (110) signale la présence d'une ou plusieurs personnes (300) dans l'espace, définit, pour au moins une de ces personnes, une zone dynamique (X) à partir des données de contour de celle-ci, à partir desquelles des informations de mouvements de la personne ou sur la personne (300) doivent être obtenues,
**en ce qu'**un deuxième module de détection est prévu, qui est conçu pour la détection de mouvements d'une personne, le deuxième module de détection recevant des informations uniquement d'une zone dynamique (X) et **en ce que**
la logique d'analyse (120) détecte un état de l'espace et/ou d'une personne (300) et/ou d'un objet à partir des signaux traités, les seules informations visuelles utilisées pour la détection de l'état étant celles qui proviennent d'une des zones, la logique d'analyse (120) déduisant des mouvements de la personne correspondante lorsque des modifications de contours de la zone dynamique (X) sont détectées, et la logique d'analyse (120) prenant une décision basée sur l'état détecté, afin de déterminer si une action doit être entreprise ou non.

2. Système selon la revendication 1, **caractérisé en ce que** le premier module de détection détecte le contour d'une personne ou d'un objet et déterminer son identité à l'aide du contour.

3. Système selon la revendication 1, **caractérisé en ce que** le premier et/ou le deuxième module de détection font partie de la logique d'analyse (120).

4. Système selon la revendication 1, **caractérisé en ce que** l'action est un message à un récepteur externe.

5. Système selon la revendication 1, **caractérisé en ce que**, pour l'enregistrement d'informations visuelles provenant de toutes les zones, une seule caméra (110) est prévue.

6. Procédé de traitement d'informations visuelles, auditives, olfactives et/ou haptiques pour la détermination cognitive d'au moins un état d'au moins un espace et/ou d'au moins une personne et/ou d'au moins un objet et pour trouver et prendre une décision en fonction de l'au moins un état détecté, comprenant les étapes suivantes :
- détermination d'au moins la présence et, le cas échéant, l'identification de personnes et/ou d'objets et l'émission d'un signal correspondant ;
- enregistrement quantitatif ou qualitatif d'informations visuelles, auditives, olfactives et/ou haptiques et émission de signaux électriques correspondants ; et
- - traitement des signaux émis ;
- définition d'une ou plusieurs zones statiques (A, B, C) pour un espace, chaque zone identifiant une partie de l'espace à partir de laquelle des informations visuelles, auditives, olfactives et/ou haptiques concernant l'état de l'espace, d'une personne et/ou d'un objet doivent être obtenues ;
**caractérisé en ce que**
- dès que la présence d'une ou plusieurs personnes (300) est détectée dans l'espace, définition d'une zone dynamique (X) pour au moins une de ces personnes à partir de données de contour de celle-ci, à partir de laquelle des informations de mouvements doivent être obtenues ;
- détection d'un état de l'espace et/ou d'une personne et/ou d'un objet à partir des signaux traités, les seules informations visuelles utilisées pour la détection de l'état étant celles qui proviennent d'une des zones (A, B, C, X) ;
- détection de mouvements d'une personne (300), seules les informations provenant de la zone dynamique (X) correspondant à cette personne (300) étant utilisées, et
- décision, sur la base de l'état détecté, si une action doit être entreprise ou non.

7. Procédé selon la revendication 6, **caractérisé en ce que**
- détermination d'un ensemble de conditions ;
- définition d'une ou plusieurs zones statiques temporaires (A, B, C) pour chaque condition de l'ensemble de conditions ;
- vérifier si au moins une condition est survenue ; et
- activation des zones statiques temporaires (A, B, C), qui sont définies pour l'au moins une condition ; et
- obtention d'informations visuelles à partir des zones statiques temporaires.

8. Procédé selon la revendication 6, **caractérisé en ce que** le contour d'une personne (300) ou d'un objet (380) est déterminé et son identité est déterminée à l'aide du contour.

9. Procédé selon la revendication 6, **caractérisé en ce que** l'action est un message à un récepteur externe.
